# EUROPEAN PATENT SPECIFICATION

(11) **EP 3 694 457 B1**
(45) Date of publication and mention of the grant of the patent: **28.10.2026**
(21) Application number: 18785773.5
(22) Date of filing: 21.09.2018
(51) Int. Cl.: A61F 13/02, A61M 1/00, A61M 25/02

(54) **SECUREMENT DRESSING WITH CONFORMAL BORDER**
FIXIERUNGSVERBAND MIT KONFORMER UMRANDUNG
PANSEMENT DE FIXATION À BORDURE CONFORMABLE

(30) Priority: 09.10.2017 US 201762569732 P
(43) Date of publication of application: 19.08.2020
(73) Proprietor: Solventum Intellectual Properties Company, Eagan, MN 55121 (US)
(72) Inventor: HEINECKE, Steven B., Saint Paul Minnesota 55133-3427 (US); JACOBSON, Richard L., Saint Paul Minnesota 55133-3427 (US); SCHEIBEL, Krystal Jo, Saint Paul Minnesota 55133-3427 (US); ZHANG, Ying, Saint Paul Minnesota 55133-3427 (US); PETERSON, Donald G., Saint Paul Minnesota 55133-3427 (US); HA, Phong V., Saint Paul Minnesota 55133-3427 (US)
(74) Representative: Mathys & Squire
(86) International application number: PCT/IB2018/057338
(87) International publication number: WO 2019/073326

(56) References cited:
- EP-A2- 0 284 219
- WO-A1-95/20415
- US-B1- 6 607 799

## Description

The present disclosure relates to a securement dressing with a conformal border and one or more tubing slots.

### BACKGROUND

Transparent film dressings are widely used as protective layers over wounds because they facilitate healing in a moist environment while acting as a barrier to contaminating liquids and bacteria. The films are also used as surgical drapes because of their barrier properties. Dressings and drapes fitting the above description are available under a number of trade names such as TEGADERM^{™} (3M Company, St. Paul, Minn.) and OP-SITE^{™} (Smith & Nephew, Hull, England).

The polymeric films used in those dressings and drapes are conformable, i.e., the films are extremely thin, flexible and supple. They are typically supplied with a releasable protective liner covering the adhesive coated surface of the film. When the liner is removed, the adhesive coated film tends to wrinkle and adhere to itself, interfering with the smooth, aseptic application of the dressing to a patient's skin. Various delivery systems have been proposed to address this problem such as those disclosed in U.S. Pat. No. 6,685,682. The use of a removable carrier, which does not require tearing of the film after it has been placed on the patient, avoids the problems described above. The carrier also aids in accurate placement of the dressing on a patient.

Thin polymeric films that are flexible and resilient are beneficial when used on skin that flexes, stretches, and retracts. However, for some applications, like when securing devices such as tubing, ports, and catheters, the high flexibility and resiliency of the thin polymeric film can cause too much movement of the secured medical device. Therefore, medical dressings have been developed that further incorporate areas having secured to the thin polymeric film, stiffer, less conformable materials such as adhesives, films, or fabrics. WO 95/20415 e.g. describes a cannula or catheter dressing comprising a body portion of flexible adherent sheet material for covering a wound site caused by the insertion of a catheter or cannula into a patient; and a handle an edge of which defines an aperture for receiving the catheter or cannula wherein the handle comprises a material less flexible than the body portion. For example, U.S. Pat. No. 5,088,483 discloses an adhesive composite that includes a conformable backing and a permanent adhesive reinforcement around the periphery of the adhesive composite. One example of a commercially available medical dressing with a reinforcement layer is TEGADERM^{™} IV Advanced Dressing (3M Company, St. Paul Minn.). US6607799 and EP0284219 also represent relevant prior art.

In some instances, medical dressings are applied to a patient and remain in place for several days. When dressings are worn over time, the edge of the dressing can begin to peel away from the patient possibly resulting in contamination at the site or adhesive failure entirely. The use of less resilient materials to add stiffness and reduce flexibility in areas of the dressing can contribute to adhesive failure of the dressing on skin. When the skin flexes and stretches, but the less resilient material cannot flex and/or stretch, then the adhesive may be more likely to pull away from the skin.

### SUMMARY

Medical dressings having flexible and conformable borders when attached to skin and devices such as, e.g., catheters, etc. to facilitate strong adhesive securement of the medical dressings to skin over extended periods of time are disclosed herein.

Broadly speaking medical dressings described herein include a relatively low modulus backing layer paired with a relatively high modulus support material, the backing layer and a tubing slot configured to, e.g., stabilize a catheter other tubing on the skin of a patient, and the backing layer extending outwardly from the support material over the perimeter of the medical dressing to form a border of adhesive material with elasticity to resist lifting from a patient's skin.

The present invention provides a medical dressing as set in claim 1, the medical dressing including: a backing layer comprising a first major surface and a second major surface opposite the first major surface, the backing layer comprising a backing perimeter that forms an outer boundary of the backing layer; an adhesive on the second major surface of the backing layer; support material secured to the backing layer, wherein the support material is less elastic than the backing layer, and wherein the support material comprises a support perimeter that forms an outer boundary of the support material, the support perimeter being spaced inwardly from the backing perimeter such that the backing layer forms a border between the support perimeter and the backing perimeter; and a tubing slot formed through the first and second major surfaces of the backing layer, wherein the tubing slot extends from a receiving end that interrupts the backing perimeter of the backing layer to a terminal end located within the backing layer, the tubing slot comprising a pair of opposing edges extending from the receiving end to the terminal end of the tubing slot, and wherein the pair of opposing edges of the tubing slot interrupt the support perimeter of the support material, and further wherein at least a portion of each opposing edge of the pair of opposing edges is coincident with a tubing slot edge of the support material. The support material is attached to either the first major surface of the backing layer or the second major surface of the backing layer.

The words "preferred" and "preferably" refer to embodiments that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments.

As used herein, "a," "an," "the," "at least one," and "one or more" are used interchangeably. The term "and/or" (if used) means one or all of the identified elements or a combination of any two or more of the identified elements.

The term "comprises" and variations thereof do not have a limiting meaning where those terms appear in the description and claims.

Also herein, the recitations of numerical ranges by endpoints include all numbers subsumed within that range (e.g., 1 to 5 includes 1, 1.5, 2, 2.75, 3, 3.80, 4, 5, etc.).

### BRIEF DESCRIPTIONS OF THE DRAWING

FIG. 1 depicts one illustrative embodiment of a medical dressing as described herein;
FIG. 2 depicts the medical dressing of FIG. 1 in position between a carrier and a release liner before use;
FIG. 3 is a side sectional view of FIG. 2 through line 3-3 in FIG. 2;
FIG. 4 depicts an illustrative configuration of a medical dressing not according to the claimed invention;
FIG. 5 depicts, in an enlarged view, another illustrative configuration of a tubing slot in a medical dressing not according to the claimed invention;
FIG. 6 depicts, in an enlarged view, another illustrative configuration of a tubing slot in a medical dressing not according to the claimed invention;
FIG. 7 depicts another illustrative configuration of a medical dressing not according to the claimed invention in which the support material does not include a window formed therein;
FIG. 8 depicts another illustrative configuration of a medical dressing not according to the claimed invention including multiple variations of tubing slots; and
FIGS. 9-11 are enlarged views of different selected tubing slots depicted in FIG. 8.

While the above-identified drawings and figures set forth embodiments of the invention, other embodiments are also contemplated, as noted in the discussion. In all cases, this disclosure presents the invention by way of representation and not limitation. It should be understood that numerous other modifications and embodiments can be devised by those skilled in the art, which fall within the scope and spirit of this invention.

The figures may not be drawn to scale.

### DESCRIPTION OF ILLUSTRATIVE EMBODIMENTS

Before any illustrative embodiments are described in detail, it is to be understood that the invention is not limited in its application to the details of construction and the arrangement of components set forth in the following description or illustrated in the following drawings. The invention is capable of other embodiments and of being practiced or of being carried out in various ways.

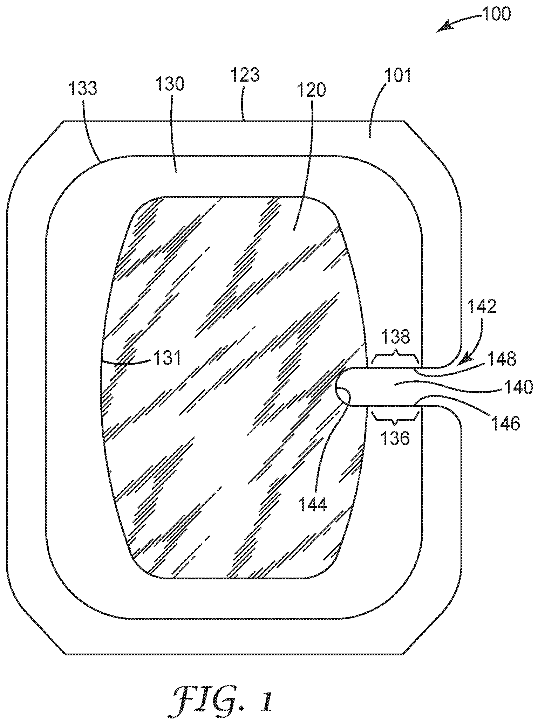

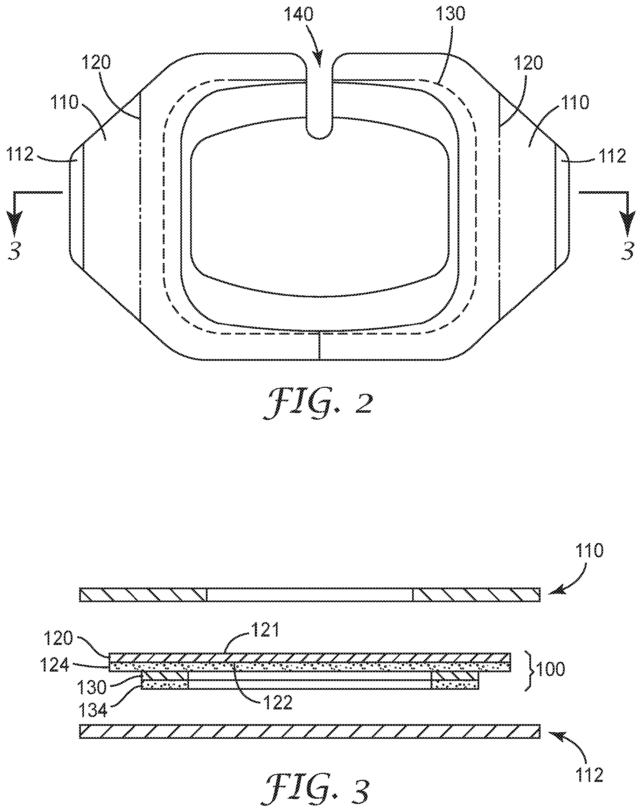

FIG. 1 depicts one major surface of one illustrative embodiment of a medical dressing 100 as described herein, while FIGS. 2-3 depict the medical device 100 located between a carrier 110 and a release liner 112 as is commonly known in connection with medical dressings. In one or more embodiments, the medical dressing 100 may be used to cover the site of an inserted intravenous catheter or other device.

The medical dressing 100 includes a backing layer 120 having a first major surface 121 and a second major surface 122 opposite the first major surface 121. The medical dressing 100 also includes adhesive 124 located on the second major surface of the backing layer 120, and a support material 130 secured to the backing layer. In the depicted illustrative embodiment, the support material 130 is secured to the second major surface 122 of the backing layer 120. In one or more embodiments, the support material 130 may include adhesive 134 on the surface of the support material 130 that faces away from the backing layer 120 such that both the backing layer 120 and the support material 130 may be adhered to the skin of a patient.

Although the support material 130 is depicted as attached to the second major surface 122 of the backing layer 120 in the depicted illustrative embodiment, in one or more alternative embodiments, the support material may be attached to the first major surface of the backing layer. In still other embodiments, support material may be attached to both the first and second major surfaces of the backing layer.

In one or more embodiments, adhesives 124 and 134 may be skin friendly adhesives useful in securing a medical dressing to the skin of a patient. In one or more embodiments, the adhesive 124 and adhesive 134 on the medical dressing 100 may be the same adhesives, while in one or more alternative embodiments, the adhesives 124 and 134 may be different. Although the adhesives 124 and 134 are depicted as solid continuous layers, it will be understood that adhesives used in connection with the medical dressings described herein may be discontinuous (e.g., pattern coated) as is commonly known (with any discontinuous coating being the same or different for adhesives on the backing layer and the support material). Further details regarding adhesives that may be used in one or more embodiments of the medical dressings described herein are discussed below.

As described above, the support material is less elastic than the backing layer.

In one or more embodiments, the backing layer 120 may be described as having a relatively high modulus of elasticity such that the backing layer 120 exhibits elasticity. Potentially suitable backing layer materials are described herein below. In contrast to the backing layer 120, the support material 130 may be described as having a relatively low modulus of elasticity such that the support material 130 exhibits less elasticity than the backing layer 120. Potentially suitable support layer materials are described herein below.

In one or more embodiments, the backing layers used in medical dressings as described herein may be both elastic and provide a sufficiently impermeable barrier to the passage of liquids and at least some gases to protect a covered site from external contaminants. Being elastic allows for the backing layer to expand, contract, stretch and recover as the underlying substrate, such as, e.g., skin, moves.

Elasticity can be measured by any number of commonly used techniques for evaluating stretch and recovery of a material. In one embodiment, the backing layer (independent of the support material) has an elongation to break of at least 200%. In one embodiment, the backing layer (independent of the support material) has an elongation to break of less than 800%. In one embodiment, the support material (independent of the backing layer) has an elongation to break of at least 20%. In one embodiment, the support material (independent of the backing layer) has an elongation to break of at least 100%. In one embodiment, the support material (independent of the backing layer) has an elongation to break less than 200%. In other embodiments, the support material (independent of the backing layer) has an elongation to break of 100% or less, 50% or less, 20% or less, or even 10% or less.

Elasticity can be measured by an initial modulus of elasticity, which is the force require to apply a specified amount of stretch. In one embodiment, the backing layer has a modulus, at 10% elongation, of less than 2 Newtons. In one embodiment, the backing layer has a modulus, at 10% elongation, of less than 1.5 Newtons. In one embodiment, when the support material is attached to the backing layer as described herein, the modulus at 10% elongation is greater than 1 Newton and less than 6 Newtons. In one embodiment, when the support material is attached to the backing layer, the modulus at 10% elongation is greater than 2 Newtons and less than 5 Newtons.

The support material may be attached to the backing layer using one or more of adhesive, thermal bonding, lamination, or other commonly used securement techniques. The support material provides structural strength to the medical dressings described herein, and, therefore, independently, is less elastic that the backing layer. In one embodiment, the support material, independently, has essentially no elasticity, such that it may be unable to stretch and/or it may be unable to recover from a stretch. In one embodiment, the support material, may be more elastic in one direction (e.g., a machine direction) than in the cross direction (e.g., a transverse direction), but overall has less elasticity than the backing layer.

Extending the backing layer in medical dressings as described herein past the support material allows for a border on medical dressings described herein that has more elasticity than portions of the medical dressing in which the support material is located. As a result, significant portions of the perimeter of a medical dressing as described herein may exhibit improved conformity to a patient's skin while still retaining the ability to provide sufficient control and/or support to, e.g., a catheter or other device over which the medical dressing is located by virtue of the support material attached to the backing layer in selected areas of the medical dressings

The backing layer 120 includes a backing perimeter 123 that forms the outer boundary of the backing layer 120. The support material 130 includes a support perimeter 133 that forms an outer boundary of the support material which is spaced inwardly from the backing perimeter 123 such that the backing layer 120 forms a border 101 (see, e.g., FIG. 1) between the support perimeter 133 and the backing perimeter 123.

In the depicted illustrative embodiment of medical dressing 100, the support material 130 further includes an inner perimeter 131 that defines a window through which the second major surface 122 of the backing layer 120 is exposed.

In illustrative medical dressing 100, a tubing slot 140 is formed through the first and second major surfaces 121 and 122 of the backing layer 120. The tubing slot 140 may be configured to receive a tube of a catheter or other device to, for example, assist in securing the catheter in position while, in one or more embodiments, other portions of the medical dressing 100 provide protection for an intravenous insertion site.

As can be seen in Fig. 1, the tubing slot 140 extends from a receiving end 142 to a terminal end 144 located within the backing layer 120. The tubing slot 140 interrupts the backing perimeter 123 at its receiving end 142 such that it may be described that the backing perimeter 123 is not continuous about the perimeter of the medical dressing 100.

As can be seen in Fig. 1, the tubing slot 140 includes a pair of opposing edges 146 and 148 that extend from the receiving end 142 of the tubing slot 140 to the terminal end 144 of the tubing slot 140. As can be seen in the depicted illustrative embodiment of medical dressing 100 the pair of opposing edges 146 and 148 interrupt the support perimeter 133, and in some embodiments, as can be seen in the depicted illustrative embodiment, the pair of opposing edges 146 and 148 may interrupt the inner perimeter 131. of the support material 130. Further, at least a portion of each opposing edge 146 and 148 is coincident with a tubing slot edge 136 and 138 of the support material 130. As a result, the support material 130 extends along portions of the opposing edges 146 and 148 to support the opposing edges 146 and 148 after removal of the medical dressing from the carrier 110 and release liner 112.

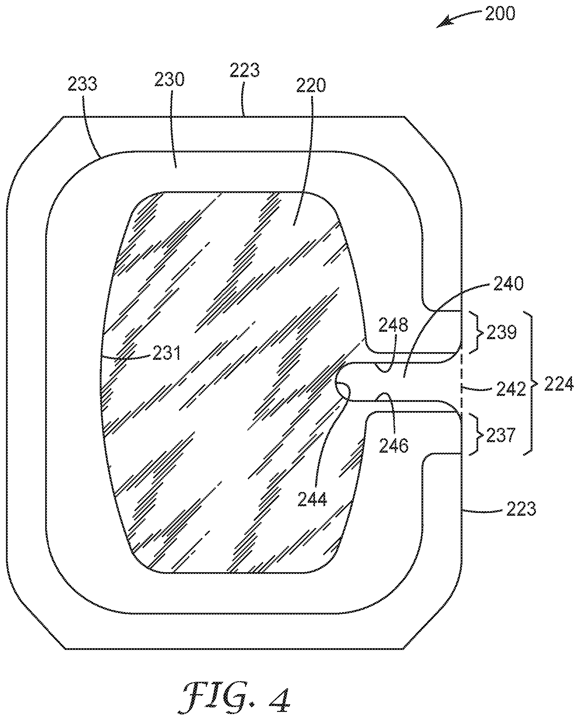

An illustrative configuration of a medical dressing not according to the claimed invention is depicted in FIG. 4 in the form of medical dressing 200. As depicted there, the medical dressing 200 includes a backing layer 220 having a backing perimeter 223 and a support material 230 having a support perimeter 233 and an inner perimeter 231 similar to that described above with respect to medical dressing 100.

Further, medical dressing 200 also includes a tubing slot 240 having a receiving end 242, a terminal end 244, along with a pair of opposing edges 246 and 248. Unlike the medical dressing 100, support material 230 of medical dressing 200 extends outwardly to the backing perimeter 223 of the backing layer 220 on opposite sides of the tubing slot 240 to provide support to the backing layer 220 during delivery and placement of the medical dressing 200 on a patient.

The receiving end 242 of the tubing slot 240 may be described as interrupting a first portion 224 of the backing perimeter 223 of the backing layer 220. The support material 230 extends outwardly towards the backing perimeter 223 to a first support material edge 237 that is coincident with a section of the first portion 224 of the backing perimeter 223 on one side of the receiving end 242 of the tubing slot 240. The support material 230 also extends outwardly toward the backing perimeter 223 to a second support material edge 239 that is coincident with a section of the first portion 224 of the backing perimeter 223 on an opposing side of the receiving end 242 of the tubing slot 240. As a result, the first and second support material edges 237 and 239 of the support material 230 are located on opposite sides of the receiving end 242 of the tubing slot 240.
In medical dressing 200, the backing perimeter 223 may be described as having a second portion that is exclusive of the first portion 224. Further, the support material 230 is spaced inwardly of that second portion of the backing perimeter 223 such that the backing layer 220 forms a border between the support perimeter 233 of the support material 230 and the second portion of the backing perimeter 223 outside of the first portion 224 of the backing perimeter 223. In medical dressing 200, the first portion of the backing perimeter (i.e., the portion of the backing perimeter along which the support material is coincident with the backing layer) may have a length that is limited such that the backing layer forms a border around a significant majority of the perimeter of the medical dressing. The first portion of the backing perimeter may be described as having a length that is equal to or less than 2, 1.5, or 1 times the tubing slot length, where the tubing slot length is measured from the receiving end of the tubing slot to its terminal end.

In medical dressing 200, the receiving end 242 of the tubing slot 240 may be located proximate a midpoint of the first portion 224 of the backing perimeter 223.

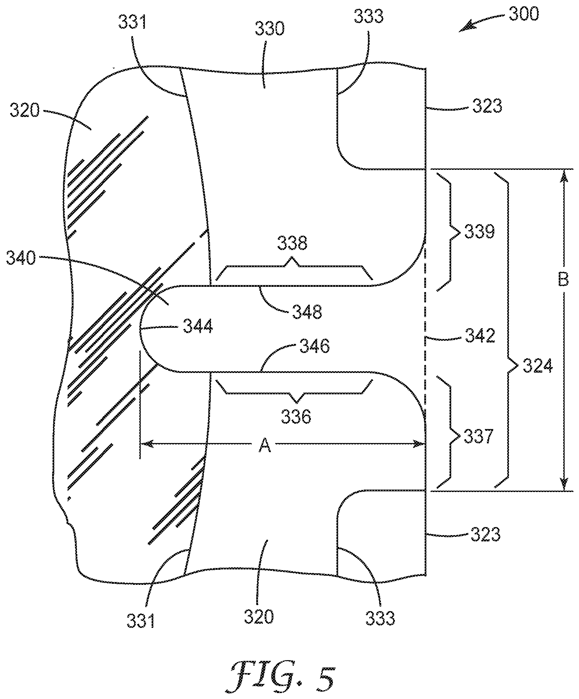

An alternative construction for a tubing slot in a medical dressing 300 - not according to the claimed invention - is depicted in FIG. 5 where the tubing slot 340 includes a pair of opposing edges 346 and 348 extending from a receiving end 342 to a terminal end of the tubing slot 340.

The medical dressing 300 includes a backing layer 320 and support material 330, with the support material 330 including a pair of opposing support material slot edges 336 and 338 that are coincident with at least a portion of the pair of opposing edges 346 and 348 of the tubing slot 340.

Further, the support material 330 also extends outwardly to the backing perimeter 323 of the backing layer 320 on opposite sides of the tubing slot 340 such that the support material 330 includes support material edges 337 and 339 that are coincident with sections of the first portion 324 of the backing perimeter 323 of the backing layer 320 of medical dressing 300.

As a result, the support material 330 in medical dressing 300 provides support along both the first portion 324 of the backing perimeter 323 as well as along the opposing edges of the tubing slot 346 and 348.

Medical dressing 300 does include an inner perimeter 331 of support material 330 such that the terminal end 344 of tubing slot 340 is not supported by the support material 330 but is, instead, formed only in backing layer 320.

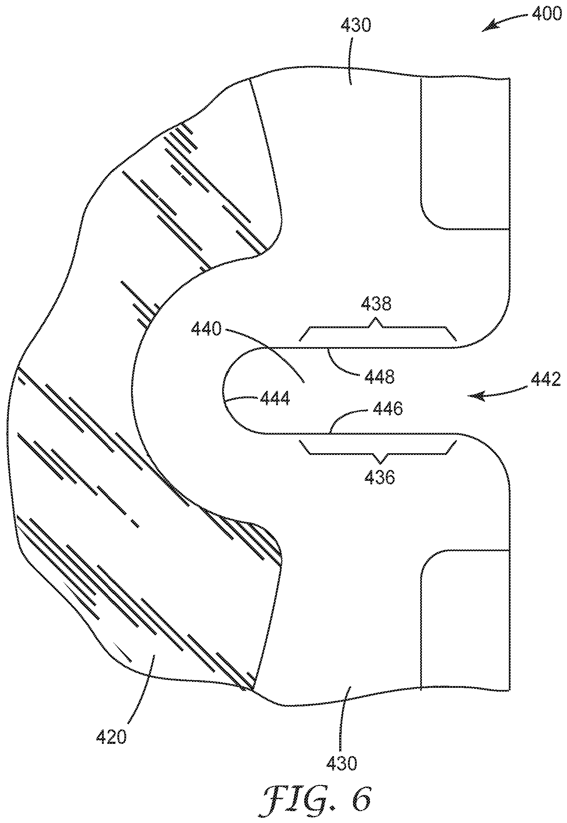

In alternative configuration of a medical dressing 400 - not according to claimed invention - as depicted in FIG. 6, the support material 430 extends inwardly of the tubing slot 440 such that the tubing slot 440 is surrounded by support material 430. In this illustrative configuration, the opposing edges 446 and 448 that extend from the receiving end 442 to the terminal end 444 of the tubing slot 440 may be described as being coincident with edges in the support material 430 along their entire lengths.

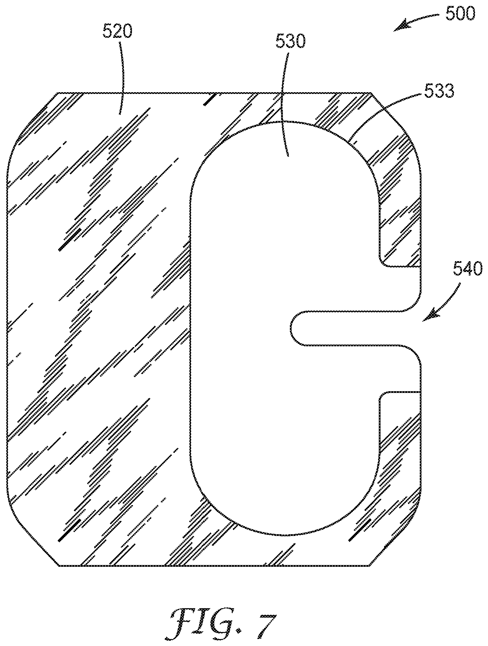

Yet another illustrative configuration of a medical dressing 500 - not according to claimed invention - is depicted in FIG. 7 and includes backing layer 520, support material 530 having a support perimeter 533, and a tubing slot 540. Unlike medical dressings 100, 200, 300, and 400, however, the support material 530 of medical dressing 500 does not include an inner perimeter forming a window as described in each of medical dressings 100, 200, 300, and 400.

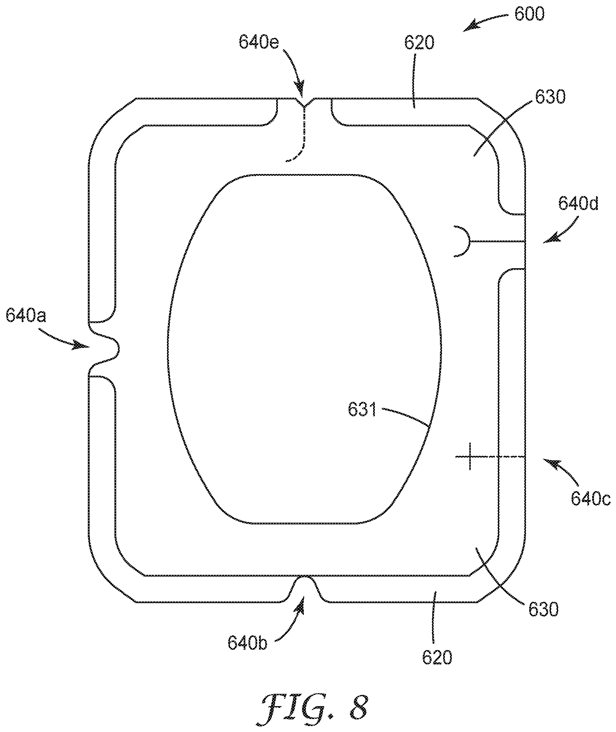

Still another illustrative configuration of a medical dressing 600 - not according to claimed invention - is depicted in FIG. 8 where medical dressing 600 includes a backing layer 620 and support material 630. Medical dressing 600 includes support material 630 having an inner perimeter 631 that defines an open window as discussed herein.

Medical dressing 600 includes a variety of different tubing slots. Medical dressings described herein may include two or more tubing slots in a single medical dressing; also tubing slots having a variety in the shape and/or configuration may be provided in medical dressings as described herein.

Returning to medical dressing 600 depicted in FIG. 8, tubing slots 640a and 640b have a shorter tubing slot length as compared to the tubing slots e.g. depicted in connection with the medical dressings of FIGS. 1-7..

As used herein, a "tubing slot" may include opposing edges that are separated from each other such that the tubing slot has a measurable width (e.g., 1 millimeter, 2 millimeters, 5 millimeters, etc.) or, alternatively, a tubing slot may be provided as a slit, cut, line of perforations, etc. formed through a backing layer (and, optionally, support material). Further, tubing slots as provided in connection with medical dressings as described herein may be straight, curved, or combinations of one or more straight and one or more curved sections. In addition, the receiving end of a tubing slot is that end of the tubing slot proximate or at the outer perimeter of the medical dressing and is typically the portion of the tubing slot that receives tubing (of, e.g., a catheter) as the dressing is positioned and secured to a patient.

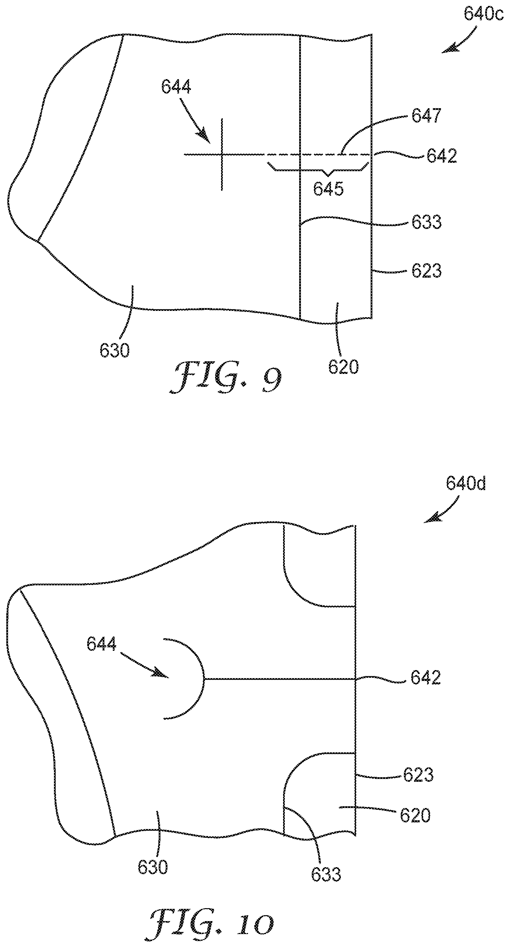

Again, returning to medical dressing 600 depicted in FIG. 8, although medical dressing 600 per se is not according to the claimed invention it is useful in understanding potential, additional variations in tubing slots that may be appropriately provided in medical dressings as described herein for example as seen in connection with tubing slots 640c, 640d, and 640e. One feature of tubing slots 640c, 640d, and 640e is in the width of the tubing slots as compared to their length. In particular, tubing slots 640c, 640d, and 640e are in the form of slits formed in the backing layer 620 and at least partially in the support material 630. Each of the tubing slots 640c, 640d, and 640e is separately depicted in enlarged views in FIGS. 9-11.

In those embodiments in which the tubing slot of a medical dressing as described herein has opposing edges that are spaced apart from each (as in, e.g., FIGS. 1-3), the tubing slot may, in one or more embodiments, have a maximum tubing slot width measured transverse to the tubing slot length that is equal to or greater than 5 percent, 10 percent, 15 percent or 20 percent of the tubing slot length.

In FIG. 9, tubing slot 640c can be seen as including a receiving end 642 and a terminal end 644. In this depicted configuration of a tubing slot - that may be appropriately used in a medical dressing as described herein - the terminal end 644 is depicted in the form of a T or cross shaped slit that maybe helpful in accommodating a tube having a larger diameter. Another optional feature depicted in connection with tubing slot 640c is that a portion 645 of the tubing slot 640c includes a series of perforations 647 extending from the receiving end 642 towards the terminal end 644 of the tubing slot 640c. The use of perforations within the border located between the backing perimeter 623 and the support material perimeter 633 may provide additional support to the backing layer 620 within the border during delivery and placement of a medical dressing as described herein.

In one or more embodiments of tubing slots in medical dressings as described herein that include perforations, the perforations may, e.g., have a perforation length of 0.2 mm to 5.0 mm, 0.5mm to 3.0 mm, or 1.0 mm to 2.0 mm, with a connecting segment (e.g., land) length of 0.1 mm 1.0 mm, 0.2 mm to 0.8 mm, 0.3mm to 0.6 mm. In other embodiments, the perforations may be described has having a perforation : land ratio of 1:1 to 10:1, 2:1 to 8:1, or 3:1 to 6:1.

Referring to FIG. 10, tubing slot 640d also includes a receiving end 642 at the backing perimeter 623 of backing layer 620 as well as a terminal end 644 located inward of the receiving end 642. Tubing slot 640d is formed entirely of continuous slits formed through the backing layer 620 as well as the support material 630 (the support material of which, in the depicted medical dressing 600/tubing slot 640d extends outwardly to the backing perimeter 623 of the backing layer 620). Another variation depicted in connection with tubing slot 640d is the generally Y-shaped terminal end 644 which may also assist in conforming the medical dressing around a tube of a catheter or other similar article.

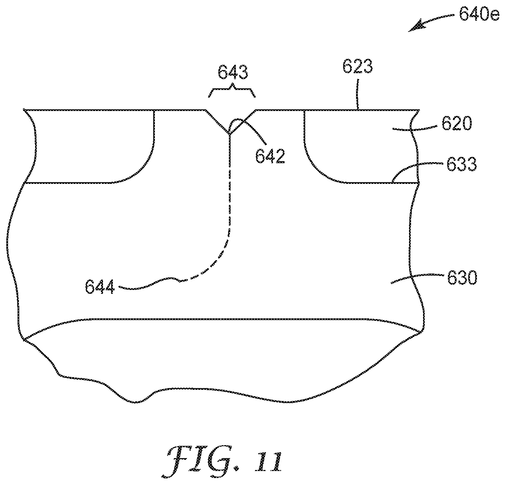

Referring to FIG. 11, tubing slot 640e is depicted in an enlarged view where the tubing slot 640e includes a receiving end 642 and a terminal end 644. As in tubing slot 640d, tubing slot 640 is formed in both the backing layer 620 and the support material 630 (again the support material of which in the depicted medical dressing 600/tubing slot 640e extends outwardly to the backing perimeter 623 such that the full length of the tubing slot 640e is supported by the support material). Variations in the tubing slot 640e include positioning of the receiving end 642 of the tubing slot 640e within a notch 643, that in case of the depicted tubing slot 640e is formed into the backing perimeter 623 of the backing layer 620 as well as the support material 630. Another variation in the tubing slot 640e is its curved shape which may assist in conforming the medical dressing around tubing of a catheter or similar article when in use. Yet another variation is that the tubing slot 640e is constructed of perforations formed in the backing layer 620 and the support material 630.

The medical dressings described herein may be made by conventional techniques (e.g., extrusion, solvent casting, calendaring, laminating, and adhesive coating) which are familiar to those skilled in the art. U.S. Pat. No. 6,685,682 discloses some potentially useful constructions and methods for making medical dressings with backing layers and support material as described herein.

### BACKING LAYERS

The backing layers of one or more embodiments of medical dressings described herein may provide an impermeable barrier to the passage of liquids and at least some gases. Representative backing layers may include non-woven and woven fibrous webs, knits, films, foams, polymeric films and other familiar backing materials. In some embodiments, a transparent backing layer is desirable to allow for viewing of the underlying skin or medical device.

In one embodiment, the backing layer has high moisture vapor permeability, but generally impermeable to liquid water so that microbes and other contaminants are sealed out from the area under the backing layer. One example of a suitable material is a high moisture vapor permeable film such as described in U.S. Pat. Nos. 3,645,835 and 4,595,001. In high moisture vapor permeable film/adhesive composites, the composite should transmit moisture vapor at a rate equal to or greater than human skin such as, for example, at a rate of at least 300 g/m²/24 hrs at 37° C./100-10% RH, or at least 700 g/m²/24 hrs at 37° C./100-10% RH, or at least 2000 g/m²/24 hrs at 37° C./100-10% RH using the inverted cup method as described in U.S. Pat. No. 4,595,001. Perforated substrates or films or pattern coated adhesives may be used to increase the moisture vapor transmission. In one embodiment, the backing layer is an elastomeric polyurethane, polyester, or polyether block amide films. These films combine the desirable properties of resiliency, elasticity, high moisture vapor permeability, and transparency. A description of this characteristic of backing layers can be found in issued U.S. Pat. Nos. 5,088,483 and 5,160,315.

Commercially available examples of potentially suitable backing layers may include the thin polymeric film backings sold under the trade names TEGADERM (3M Company), OPSITE (Smith & Nephew), etc. Many other backing layers may also be used, including those commonly used in the manufacture of surgical incise drapes (e.g., incise drapes manufactured by 3M Company under the trade names STERIDRAPE and IOBAN), etc.

Because fluids may be actively removed from the sealed environments defined by the medical dressings, a relatively high moisture vapor permeable backing layer may not be required. As a result, some other potentially useful backing materials may include, e.g., metallocene polyolefins and SBS and SIS block copolymer materials could be used.

Regardless, however, it may be desirable that the backing layer be kept relatively thin to, e.g., improve conformability. For example, the backing layer may be formed of polymeric films with a thickness of 200 micrometers or less, or 100 micrometers or less, potentially 50 micrometers or less, or even 25 micrometers or less.

### SUPPORT MATERIALS

The support materials used in one or more embodiments of medical dressings as described herein may provide strength to the backing layer. The support material therefore has more stiffness and less elasticity than the backing layer. The support material may be a coating, such as an adhesive, or may be a self-supporting substrate such as another film, woven, knitted, or nonwoven fabric. For example, U.S. Pat. No. 5,088,483 discloses a permanent adhesive as a reinforcement that could be used as the support material.

One example of nonwoven for the support material is a high strength nonwoven fabric available from E. I. Dupont de Nemours & Company of Wilmington, Del. under the trademark Sontara, including Sontara 8010, a hydroentangled polyester fabric. Other suitable nonwoven webs include a hydroentangled polyester fabric available from Veratec, a division of International Paper of Walpole, Mass. Another suitable nonwoven web is the nonwoven elastomeric web described in U.S. Pat. No. 5,230,701.

### ADHESIVES

Suitable adhesive for use in one or more embodiments of the medical dressings described herein include any adhesive that provides acceptable adhesion to skin and is acceptable for use on skin (e.g., the adhesive should preferably be non-irritating and non-sensitizing). Suitable adhesives are pressure sensitive and in certain embodiments have a relatively high moisture vapor transmission rate to allow for moisture evaporation. Suitable pressure sensitive adhesives include those based on acrylates, urethane, hydrogels, hydrocolloids, block copolymers, silicones, rubber based adhesives (including natural rubber, polyisoprene, polyisobutylene, butyl rubber etc.) as well as combinations of these adhesives. The adhesive component may contain tackifiers, plasticizers, rheology modifiers as well as active components including for example an antimicrobial agent.

The pressure sensitive adhesives that may be used in the medical dressings may include adhesives that are typically applied to the skin such as the acrylate copolymers described in U.S. Pat. No. RE 24,906, particularly a 97:3 isooctyl acrylate:acrylamide copolymer. Another example may include a 70:15:15 isooctyl acrylate: ethyleneoxide acrylate:acrylic acid terpolymer, as described in U.S. Pat. No. 4,737,410 (Example 31). Other potentially useful adhesives are described in U.S. Pat. Nos. 3,389,827; 4,112,213; 4,310,509; and 4,323,557. Inclusion of medicaments or antimicrobial agents in the adhesive is also contemplated, as described in U.S. Pat. Nos. 4,310,509 and 4,323,557.

Silicone adhesive can also be used. Generally, silicone adhesives can provide suitable adhesion to skin while gently removing from skin. Suitable silicone adhesives are disclosed in PCT Publications WO2010/056541 and WO2010/056543.

The pressure sensitive adhesives may, in some embodiments, transmit moisture vapor at a rate greater to or equal to that of human skin. While such a characteristic can be achieved through the selection of an appropriate adhesive, it is also contemplated that other methods of achieving a high relative rate of moisture vapor transmission may be used, such as pattern coating the adhesive on the backing, as described in U.S. Pat. No. 4,595,001. Other potentially suitable pressure sensitive adhesives may include blown-micro-fiber (BMF) adhesives such as, for example, those described in U.S. Pat. No. 6,994,904. The pressure sensitive adhesive used in the wound dressing may also include one or more areas in which the adhesive itself includes structures such as, e.g., the microreplicated structures described in U.S. Pat. No. 6,893,655.

Issued U.S. Pat. Nos. 3,645,835 and 4,595,001, describe methods of making such films and methods for testing their permeability. Preferably, the film/adhesive composite should transmit moisture vapor at a rate equal to or greater than human skin. Preferably, the adhesive coated film transmits moisture vapor at a rate of at least 300 g/m²/24 hrs/37 C/100-10% RH, more preferably at least 700 g/m²/24 hrs/37 C/100-10% RH, and most preferably at least 2000 g/m²/24 hrs/37 C/100-10% RH using the inverted cup method as described in U.S. Pat. No. 4,595,001.

Different portions of the medical dressings described herein may include different adhesives, such as disclosed in US 2015/0141949 titled "Medical Dressing with Multiple Adhesives." For example, a portion may include an acrylate adhesive while another portion may include a silicone adhesive. In one embodiment, to prevent edge separation, adjacent the perimeter is acrylate adhesive, while near the central portion there is silicone adhesive. In one embodiment, to strongly secure with a device or tubing near the central portion there is acrylate adhesive, while near the perimeter in contact with skin is silicone adhesive.

### OPTIONAL COMPONENTS

An absorbent material may also be used in conjunction with the medical dressings described herein. An absorbent material may be the same as the wound packing material (described below) or may be a separate element. The absorbent materials can be manufactured of any of a variety of materials including, but not limited to, woven or nonwoven cotton or rayon. Absorbent pad is useful for containing a number of substances, optionally including antimicrobial agents, drugs for transdermal drug delivery, chemical indicators to monitor hormones or other substances in a patient, etc.

The absorbent may include a hydrocolloid composition, including the hydrocolloid compositions described in U.S. Pat. Nos. 5,622,711 and 5,633,010. The hydrocolloid absorbent may comprise, for example, a natural hydrocolloid, such as pectin, gelatin, or carboxymethylcellulose (CMC) (Aqualon Corp., Wilmington, Del.), a semi-synthetic hydrocolloid, such as cross-linked carboxymethylcellulose (X4ink CMC) (e.g. Ac-Di-Sol; FMC Corp., Philadelphia, Pa.), a synthetic hydrocolloid, such as cross-linked polyacrylic acid (PAA) (e.g., CARBOPOL^{™} No. 974P; B.F. Goodrich, Brecksville, Ohio), or a combination thereof. Absorbent materials may also be chosen from other synthetic and natural hydrophilic materials including polymer gels and foams.

And optional release liners may be included that covers all or a portion of the adhesives to prevent contamination of the adhesives. In one embodiment, the package that contains the adhesive dressing may serve as a release liner. Suitable release liners can be made of kraft papers, polyethylene, polypropylene, polyester or composites of any of these materials. In one embodiment, the liners are coated with release agents such as fluorochemicals or silicones. For example, U.S. Pat. No. 4,472,480 describes low surface energy perfluorochemical liners. In one embodiment, the liners are papers, polyolefin films, or polyester films coated with silicone release materials.

An optional carrier may be included that covers all or a portion of the first major surface of the substrate, providing structural support if the dressing is thin and highly flexible. The carrier maybe removable from the first major surface once the adhesive dressing is placed on skin. The carrier can be constructed of a variety of materials such as fabric that are woven or kitted, nonwoven material, papers, or film. In one embodiment, the carrier is along the perimeter of the first major surface of the dressing and is removable from the first major surface, similar to the carrier used the 3M Tegaderm^{™} Transparent Film Dressing, available from 3M Company, St. Paul, Minn.

An optional antimicrobial component may be included that is either separate from the adhesive dressing or may be integral with the dressing. The antimicrobial component is placed near or adjacent to the insertion site of the medical device to inhibit microbial growth in and around the insertion site. The antimicrobial component can be absorbent foam or gel, such as used in a 3M Tegaderm^{™} CHG I.V. Securement Dressing, available from 3M Company.

All headings are for the convenience of the reader and should not be used to limit the meaning of the text that follows the heading, unless so specified.

Although specific embodiments of this invention have been shown and described herein, it is understood that these embodiments are merely illustrative of the many possible specific arrangements that can be devised in application of the principles of the invention. Numerous and varied other arrangements can be devised in accordance with these principles by those of ordinary skill in the art without departing from the scope of the invention. Thus, the scope of the present invention should not be limited to the structures described in this application, but only by the structures described by the language of the claims.

## Claims

1. A medical dressing (100) comprising:
a backing layer (120) comprising a first major surface (121) and a second major surface (122) opposite the first major surface, the backing layer comprising a backing perimeter (123) that forms an outer boundary of the backing layer;
an adhesive (124) on the second major surface of the backing layer;
and
a tubing slot (140) formed through the first and second major surfaces of the backing layer, wherein the tubing slot extends from a receiving end (142) that interrupts the backing perimeter (123) of the backing layer to a terminal end (144) located within the backing layer, the tubing slot comprising a pair of opposing edges (146, 148) extending from the receiving end to the terminal end of the tubing slot;
**characterized in that**
the medical dressing further comprises support material (130) secured to the backing layer,
wherein the support material is less elastic than the backing layer, wherein the support material comprises a support perimeter (133) that forms an outer boundary of the support material, the support perimeter (133) being spaced inwardly from the backing perimeter (123) such that the backing layer forms a border (101) between the support perimeter and the backing perimeter, and
wherein the support material is attached to either the first major surface of the backing layer or the second major surface of the backing layer; and
further **in that** the pair of opposing edges (146, 148) of the tubing slot (140) interrupt the support perimeter (133) of the support material, and further wherein at least a portion of each opposing edge of the pair of opposing edges is coincident with a tubing slot edge (136, 138) of the support material.

2. The medical dressing (100) of claim 1, wherein the tubing slot (140) comprises a first tubing slot and further wherein the medical dressing comprises a second tubing slot, wherein the second tubing slot is formed through the first and second major surfaces of the backing layer (120), wherein the second tubing slot extends from a receiving end that interrupts the backing perimeter (123) of the backing layer to a terminal end located within the backing layer, the second tubing slot comprising a pair of opposing edges extending from the receiving end to the terminal end of the second tubing slot.

3. The medical dressing (100) of claim 1 or 2, wherein the tubing slot (140) comprises a slit formed through the backing layer (122).

4. The medical dressing (100) of claim 1 or 2, wherein the tubing slot (140) comprises a plurality of perforations formed through the backing layer (122).

5. The medical dressing (100) of claim 1 or 2, wherein the tubing slot (140) comprises a maximum tubing slot width measured transverse to the tubing slot length that is equal to or greater than 5 percent of the tubing slot length.

6. The medical dressing (100) of any one of the preceding claims, wherein the support material (130) comprises an inner perimeter (131) defining a window within the support material, wherein the backing layer (120) extends over the window.

7. The medical dressing of any one of the preceding claims, wherein the medical dressing (100) further comprises support material adhesive (134) on a surface of the support material (130) that faces away from the backing layer.

8. The medical dressing of claim 7, wherein the support material (130) is attached to the second major surface (122) of the backing layer (120), and wherein the support material adhesive (134) is the same as the adhesive (124) on the second major surface (122) of the backing layer (120).

9. The medical dressing (100) of any one of the preceding claims, wherein the backing layer (122) is transparent.

10. The medical dressing (100) of any one of the preceding claims, wherein the backing layer (122) is impermeable to liquid and can transmit moisture vapor at a rate of at least 300 g/m²/24 hrs at 37° C./100-10% RH.

## Patentansprüche

1. Ein medizinischer Verband (100), aufweisend:
eine Trägerschicht (120), aufweisend eine erste Hauptoberfläche (121) und eine zweite Hauptoberfläche (122) gegenüber der ersten Hauptoberfläche, die Trägerschicht aufweisend einen Trägerumfang (123), der eine äußere Begrenzung der Trägerschicht ausbildet;
einen Klebstoff (124) auf der zweiten Hauptoberfläche der Trägerschicht;
und
ein Schlauchschlitz (140), der durch die erste und die zweite Hauptoberfläche der Trägerschicht hindurch ausgebildet ist, wobei sich der Schlauchschlitz von einem Aufnahmeende (142), das den Trägerumfang (123) der Trägerschicht unterbricht, zu einem Abschlussende (144) erstreckt, das sich innerhalb der Trägerschicht befindet, der Schlauchschlitz aufweisend ein Paar von gegenüberliegenden Kanten (146, 148), die sich von dem Aufnahmeende zu dem Abschlussende des Schlauchschlitzes erstrecken;
**dadurch gekennzeichnet, dass**
der medizinische Verband ferner Stützmaterial (130) aufweist, das an der Trägerschicht gesichert ist,
wobei das Stützmaterial weniger elastisch als die Trägerschicht ist, wobei das Stützmaterial einen Stützumfang (133) aufweist, der eine äußere Begrenzung des Stützmaterials ausbildet, wobei der Stützumfang (133) von dem Trägerumfang (123) derart nach innen beabstandet ist, dass die Trägerschicht einen Rand (101) zwischen dem Stützumfang und dem Trägerumfang ausbildet, und
wobei das Stützmaterial entweder an der ersten Hauptoberfläche der Trägerschicht oder an der zweiten Hauptoberfläche der Trägerschicht befestigt ist; und
ferner dadurch, dass das Paar von gegenüberliegenden Kanten (146, 148) des Schlauchschlitzes (140) den Stützumfang (133) des Stützmaterials unterbricht, und ferner wobei mindestens ein Abschnitt jeder gegenüberliegenden Kante des Paares von gegenüberliegenden Kanten mit einer Schlauchschlitzkante (136, 138) des Stützmaterials zusammenfällt.

2. Der medizinische Verband (100) nach Anspruch 1, wobei der Schlauchschlitz (140) einen ersten Schlauchschlitz aufweist und wobei ferner der medizinische Verband einen zweiten Schlauchschlitz aufweist, wobei der zweite Schlauchschlitz durch die erste und die zweite Hauptoberfläche der Trägerschicht (120) hindurch ausgebildet ist, wobei sich der zweite Schlauchschlitz von einem Aufnahmeende, das den Trägerumfang (123) der Trägerschicht unterbricht, zu einem Abschlussende erstreckt, das sich innerhalb der Trägerschicht befindet, der zweite Schlauchschlitz aufweisend ein Paar von gegenüberliegenden Kanten, die sich von dem Aufnahmeende zu dem Abschlussende des zweiten Schlauchschlitzes erstrecken.

3. Der medizinische Verband (100) nach Anspruch 1 oder 2, wobei der Schlauchschlitz (140) einen Spalt aufweist, der durch die Trägerschicht (122) hindurch ausgebildet ist.

4. Der medizinische Verband (100) nach Anspruch 1 oder 2, wobei der Schlauchschlitz (140) eine Mehrzahl von Perforationen aufweist, die durch die Trägerschicht (122) hindurch ausgebildet sind.

5. Der medizinische Verband (100) nach Anspruch 1 oder 2, wobei der Schlauchschlitz (140) eine maximale Schlauchschlitzbreite, gemessen quer zu der Schlauchschlitzlänge, aufweist, die gleich oder größer als 5 Prozent der Schlauchschlitzlänge ist.

6. Der medizinische Verband (100) nach einem der vorstehenden Ansprüche, wobei das Stützmaterial (130) einen inneren Umfang (131) aufweist, der ein Fenster innerhalb des Stützmaterials definiert, wobei sich die Trägerschicht (120) über das Fenster erstreckt.

7. Der medizinische Verband nach einem der vorstehenden Ansprüche, wobei der medizinische Verband (100) ferner Stützmaterialklebstoff (134) auf einer Oberfläche des Stützmaterials (130) aufweist, die von der Trägerschicht abgewandt ist.

8. Der medizinische Verband nach Anspruch 7, wobei das Stützmaterial (130) an der zweiten Hauptoberfläche (122) der Trägerschicht (120) befestigt ist, und wobei der Stützmaterialklebstoff (134) der gleiche wie der Klebstoff (124) auf der zweiten Hauptoberfläche (122) der Trägerschicht (120) ist.

9. Der medizinische Verband (100) nach einem der vorstehenden Ansprüche, wobei die Trägerschicht (122) transparent ist.

10. Der medizinische Verband (100) nach einem der vorstehenden Ansprüche, wobei die Trägerschicht (122) flüssigkeitsundurchlässig ist und Feuchtigkeitsdampf mit einer Rate von mindestens 300 g/m²/24 Stunden bei 37 °C/100-10 % RH durchlassen kann.

## Revendications

1. Pansement médical (100) comprenant :
une couche de support (120) comprenant une première surface principale (121) et une seconde surface principale (122) opposée à la première surface principale, la couche de support comprenant un périmètre de support (123) qui forme une limite extérieure de la couche de support ;
un adhésif (124) sur la seconde surface principale de la couche de support ;
et
une fente de tube (140) formée à travers les première et seconde surfaces principales de la couche de support, dans lequel la fente de tube s'étend depuis une extrémité de réception (142) qui interrompt le périmètre de support (123) de la couche de support jusqu'à une extrémité terminale (144) située à l'intérieur de la couche de support, la fente de tube comprenant une paire de bords opposés (146, 148) s'étendant depuis l'extrémité de réception jusqu'à l'extrémité terminale de la fente de tube ;
**caractérisé en ce que**
le pansement médical comprend en outre un matériau de soutien (130) fixé à la couche de support,
dans lequel le matériau de soutien est moins élastique que la couche de support, dans lequel le matériau de soutien comprend un périmètre de soutien (133) qui forme une limite extérieure du matériau de soutien, le périmètre de soutien (133) étant espacé du périmètre de support (123) vers l'intérieur de sorte que la couche de support forme une bordure (101) entre le périmètre de soutien et le périmètre de support, et
dans lequel le matériau de soutien est fixé soit à la première surface principale de la couche de support, soit à la seconde surface principale de la couche de support ; et
en outre **en ce que** la paire de bords opposés (146, 148) de la fente de tube (140) interrompt le périmètre de soutien (133) du matériau de soutien, et en outre dans lequel au moins une partie de chaque bord opposé de la paire de bords opposés coïncide avec un bord de fente de tube (136, 138) du matériau de soutien.

2. Pansement médical (100) selon la revendication 1, dans lequel la fente de tube (140) comprend une première fente de tube et en outre dans lequel le pansement médical comprend une seconde fente de tube, dans lequel la seconde fente de tube est formée à travers les première et seconde surfaces principales de la couche de support (120), dans lequel la seconde fente de tube s'étend depuis une extrémité de réception qui interrompt le périmètre de support (123) de la couche de support jusqu'à une extrémité terminale située à l'intérieur de la couche de support, la seconde fente de tube comprenant une paire de bords opposés s'étendant depuis l'extrémité de réception jusqu'à l'extrémité terminale de la seconde fente de tube.

3. Pansement médical (100) selon la revendication 1 ou 2, dans lequel la fente de tube (140) comprend une encoche formée à travers la couche de support (122).

4. Pansement médical (100) selon la revendication 1 ou 2, dans lequel la fente de tube (140) comprend une pluralité de perforations formées à travers la couche de support (122).

5. Pansement médical (100) selon la revendication 1 ou 2, dans lequel la fente de tube (140) comprend une largeur maximale de fente de tube mesurée transversalement à la longueur de fente de tube qui est égale ou supérieure à 5 pour cent de la longueur de fente de tube.

6. Pansement médical (100) selon l'une quelconque des revendications précédentes, dans lequel le matériau de soutien (130) comprend un périmètre interne (131) définissant une fenêtre à l'intérieur du matériau de soutien, dans lequel la couche de support (120) s'étend sur la fenêtre.

7. Pansement médical selon l'une quelconque des revendications précédentes, dans lequel le pansement médical (100) comprend en outre un adhésif de matériau de soutien (134) sur une surface du matériau de soutien (130) qui est opposée à la couche de support.

8. Pansement médical selon la revendication 7, dans lequel le matériau de soutien (130) est fixé à la seconde surface principale (122) de la couche de support (120), et dans lequel l'adhésif de matériau de soutien (134) est identique à l'adhésif (124) sur la seconde surface principale (122) de la couche de support (120).

9. Pansement médical (100) selon l'une quelconque des revendications précédentes, dans lequel la couche de support (122) est transparente.

10. Pansement médical (100) selon l'une quelconque des revendications précédentes, dans lequel la couche de support (122) est imperméable aux liquides et peut transmettre la vapeur d'eau à un taux d'au moins 300 g/m²/24 heures à 37 °C/100-10 % HR.
